(19) Europäisches Patentamt
European Patent Office
Office européen des brevets

(11) Veröffentlichungsnummer : **0 002 800 B2**

(12) **NEUE EUROPÄISCHE PATENTSCHRIFT**

(45) Veröffentlichungstag der neuen Patentschrift : **09.10.91 Patentblatt 91/41**

(51) Int. Cl.$^5$ : **C07D 263/58**, C07D 277/68, A01N 31/08

(21) Anmeldenummer : **78101792.6**

(22) Anmeldetag : **20.12.78**

# (54) Optisch aktive alpha-Phenoxypropionsäure Derivate.

Die Akte enthält technische Angaben, die nach dem Eingang der Anmeldung eingereicht wurden und die nicht in dieser Patentschrift enthalten sind.

(30) Priorität : **24.12.77 DE 2758002**

(43) Veröffentlichungstag der Anmeldung : **11.07.79 Patentblatt 79/14**

(45) Bekanntmachung des Hinweises auf die Patenterteilung : **02.12.81 Patentblatt 81/48**

(45) Bekanntmachung des Hinweises auf die Entscheidung über den Einspruch : **09.10.91 Patentblatt 91/41**

(84) Benannte Vertragsstaaten : **BE DE FR GB IT NL SE**

(56) Entgegenhaltungen :
**EP-A- 0 001 473**
**EP-A- 0 002 925**
**DE-A- 2 623 558**
**DE-A- 2 650 434**
**DE-C- 1 543 841**
**Wegler "Chemie der Pflanzenschutz- und Schädlingsbekämpfungsmittel", Bd. 2 (1970) S. 278 sowie Bd. 5 (1977) S. 188-189**
**J. Agric Food Chem. 23 (1975) S. 1008-1010**

(56) Entgegenhaltungen :
**Ann. Appl. Biol. 39 (1952), 295-307**
**CHEMICAL ABSTRACTS vol. 68, no. 9, 1968, Columbus, Ohio, US; B. ABERG "Plant growth regulators. XXII. Optically active alpha-alkyl-phenoxyacetic acids" Seite 3717, Spalte 2, Abstract Nr. 38 410a**

(73) Patentinhaber : **HOECHST AKTIENGESELLSCHAFT**
**Postfach 80 03 20**
**W-6230 Frankfurt am Main 80 (DE)**

(72) Erfinder : **Nestler, Hans Jürgen, Dr.**
**Steinweg 15**
**W-6240 Königstein/Taunus (DE)**
Erfinder : **Hörlein, Gerhard, Dr.**
**Assmannshäuser Weg 23**
**W-6000 Frankfurt/Main (DE)**
Erfinder : **Handte, Reinhard, Dr.**
**Breckenheimer Strasse 45**
**W-6238 Hofheim am Taunus (DE)**
Erfinder : **Bieringer, Hermann, Dr.**
**Eichenweg 26**
**W-6239 Eppstein/Taunus (DE)**
Erfinder : **Schwerdtle, Friedhelm, Dr.**
**Oestricher Weg 5**
**W-6000 Frankfurt/Main (DE)**
Erfinder : **Langelüddeke, Peter, Dr.**
**Nelkenweg 5**
**W-6238 Hofheim am Taunus (DE)**
Erfinder : **Frisch, Peter, Dr.**
**Griesheimer Stadtweg 11**
**W-6000 Frankfurt/Main (DE)**

EP 0 002 800 B2

## Beschreibung

Optisch aktive α-Phenoxypropionsäure-Derivate

Es ist bekannt, daß viele Naturstoffe mit biologischer Wirksamkeit infolge des Vorhandenseins einer oder mehrerer asymmetrischer Kohlenstoffatome optisch aktiv sind, d.h. die Ebene des polarisierten Lichtes nach rechts (+) oder links (-) drehen. Sehr oft weisen diese Verbindungen eine höhere biologische Wirksamkeit auf als entsprechende synthetisch gewonnene, optisch inaktive Verbindungen Ähnliches gilt für manche synthetisch hergestellte Wirkstoffe wie Pharmazeutika oder Pflanzenschutzmittel mit asymmetrischen C-Atomen, die gewöhnlich bei der Synthese als optisch inaktive Racemate, d.h Gemische aus gleichen Anteilen der rechts- und links-drehenden Enantiomeren, anfallen. Auch hier wird bei der Isomerentrennung nicht seiten gefunden, daß die Wirksamkeit eines der beiden Enantiomeren höher ist als die des Racemats. Ob die links (-)- oder die rechts (+)-drehende Form die jeweils aktive ist und ob überhaupt ein Zusammenhang zwischen Wirkung und optischer Aktivität besteht, läßt sich jedoch nicht vorhersagen.

Aus der Klasse der p-substituierten α-Phenoxy-propionsäure-Derivate wurden in letzter Zeit Verbindungen bekannt, die wegen ihrer spezifisch grasherbiziden Wirkung von Interesse sind [z.B. DE-A-22 23 894, DE-A-24 33 067, DE-A-25 31 643, DE-A-26 17 804, DE-A-26 23 558, DE-A-25 46 251 DE-A-24 17 487 und DE-A-26 01 548]. Diese besitzen in Nachbarstellung zur Carbonylfunktion ein asymmetrisches C-Atom, sodaß 2 enantiomere, optisch aktive Formen existieren, die gewöhnlich als D-und L-Form bezeichnet werden.

In DE-A-2 546 251 und EP-A-483 sind unter anderem substituierte Pyridyloxyphenoxypropionsäurealkylester in racemischer Form als Herbizide beschrieben bzw. vorgeschlagen worden. Aus DE-A-2 623 558 sind Phenoxyphenoxypropionsäurederivate in racemischer Form als Herbizide bekannt. In DE-A-2 531 643 ist unter anderem die D-Form des Herbizids 2- [4-(4-Trifluormethylphenoxy)-phenoxy]propionsäure beschrieben. Aus dieser Veröffentlichung ist weiterhin bekannt, daß die D- und L-Isomeren und die racemische Mischung im Falle von 2-[4-(4-Trifluormethyl-halogenophenoxy)-phenoxy] alkancarbonsäurederivaten herbizide Aktivität besitzen, wobei die D-Form im allgemeinen die höchste und die L-Form die niedrigste Aktivität besitzt.

Weiterhin sind in den nicht vorveröffentlichten EP-A-2925 und EP-A-1473 bereits optisch aktive Pyridyloxyphenoxyalkancarbonsäurederivate als Herbizide mit im Vergleich zu den Racematen erhöhter herbizider Wirkung beschrieben.

Aus Ann. Appl. Biol. 39 (1952), 295-307 sind Untersuchungen zur Wirkung pflanzenwachstumsregulierender optisch aktiver Halogenophenoxypropionsäuren bekannt.

Es wurde nun gefunden, daß die D-Enantiomeren der Verbindungen Ia und Ib sich durch eine erheblich höhere herbizide Wirksamkeit im Vergleich zu den Racematen auszeichnen.

Gegenstand der Erfindung sind somit D-(α-Phenoxy-propionsäurederivate der Formel Ia und Ib

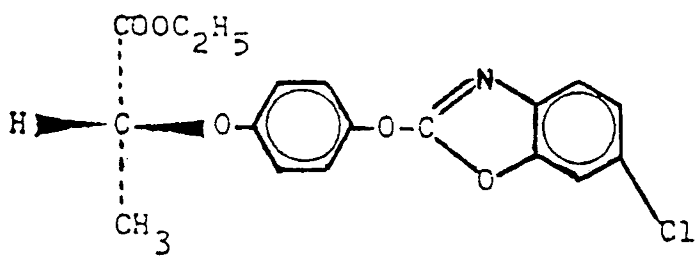

(Ia)

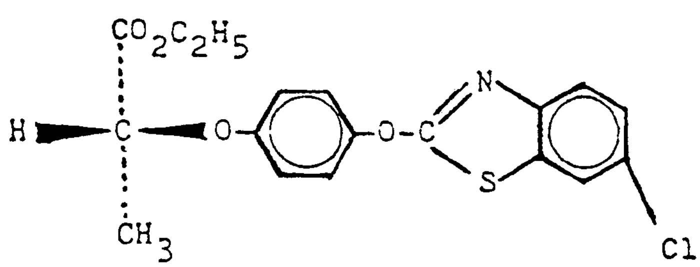

(Ib)

mit einem Gehalt an mindestens 80 % D-Form.

Man erhält die genannten Verbindungen, indem man

a) entsprechend substituierte Phenole oder Phenolate der allgemeinen Formel

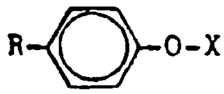

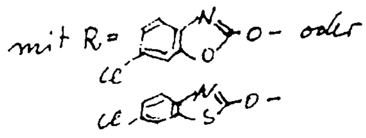


(VII)

in der X ein Alkaliatom oder Wasserstoff bedeutet, mit substituierten L-Propionsäureäthylestern der allgemeinen Formel

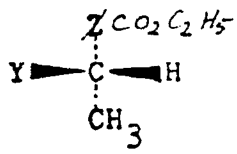

(VIII)

in der Y Chlor, Brom oder eine Sulfonyloxygruppe bedeutet oder
b) auch Verbindungen der Formel

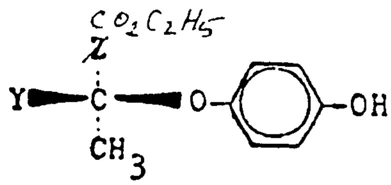

(IX)

mit einem entsprechend substituierten 2-Halogen, -benzthiazol oder -benzoxazol umsetzt.

Bei dem erfindungsgemäßen, Verfahren nach a) findet eine Waldensche Umkehrung statt, wobei die L-Konfiguration des Propionsäurederivates in die D-Konfiguration des Endproduktes übergeht.

a) zur Durchführung des verfahrens a) bedient man sich allgemein bekannter Verfahrensbedingungen. Bei Verwendung eines Phenols als Ausgangsmaterial (X = H) arbeitet man bevorzugt in Gegenwart eines Alkalicarbonates als Säurefänger und in einem polaren Lösungsmittel, vorzugsweise Aceton, Methyläthylketon, Acetonitril, Dimethylformamid oder Dimethylsulfoxyd bei Temperaturen zwischen 50 und 150°C. Verwendet man als Ausgangsverbindung der Formel VII ein Phenolat (X = Alkaliatom, vorzugsweise Na oder K), so empfiehlt sich die Anwendung von hochsiedenden Lösungsmitteln wie Toluol, Xylol, DMF oder DMSO und von Temperaturen von 100-150°C. Unter der Sulfonyloxygruppe in Y ist die Gruppe $R_{10}$-$SO_2O$- zu verstehen, in der $R_{10}$ einen aliphatischen oder aromatischen Rest bedeutet, vorzugsweise der Mesylatrest ($CH_3SO_2O$-), der Rest $CF_3SO_2O$-, der Benzolsulfonatrest, der Tosylatrest (p-$CH_3$-$C_6H_4$-$SO_2O$-) oder ein durch $NO_2$ oder $OCH_3$ substituierter Benzolsulfonatrest.

b) Die Umsetzung gemäß b) verläuft unter den gleichen Bedingungen wie a). Die Ausgangsverbindungen der Formel IX erhält man aus Hydrochinon-monobenzyläther bzw. dessen Alkalisalzen der Formel

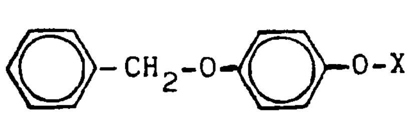

(X)

durch Umsetzung mit Verbindungen der Formel VIII und hydrogenolytische Abspaltung der Benzylgruppe. Als Katalysatoren eignen sich besonders Edelmetallkatalysatoren wie Palladium/Tierkohle.

Bei Verwendung von optisch reinem Ausgangsmaterial liefern die erfindungsgemäßen Verfahren Endprodukte mit einer optischen Reinheit von mindestens 60 % entsprechend einem Anteil von 80 % der D-Form. Wenn gewünscht, kann die optische Reinheit der Verbindungen nach üblichen Verfahren, z.B. Umkristallisation weiter erhöht werden. Falls diese Verbindungen, insbesondere die Ester, flüssig sind, ist es ein bevorzugtes Reinigungsverfahren, die zunächst erhaltenen Ester zur entsprechenden Phenoxypropionsäure zu verseifen, diese in an sich bekannter Weise durch Umkristallisation von etwa vorhandenen geringen Mengen der L-Form zu reinigen und dann aus der weitgehend reinen Säure nach einem der vorstehend angegebenen Verfahren die gewünschten Verbindungen der Formel I herzustellen.

Wie eingangs erwähnt, zeichnen sich die erfindungsgemäßen D-Enantiomeren gegenüber den Racematen durch eine erheblich gesteigerte herbizide Wirkung aus. Überraschenderweise zeigte sich, daß die L-Enantiomeren im Nachauflauf praktisch unwirksam sind. Die Wirksamkeit der Racemate beruht somit praktisch allein auf ihrem Gehalt an D-Enantiomeren.

Gegenstand der Erfindung sind somit auch herbizide Mittel, die gekennzeichnet sind durch einen Gehalt an einem Wirkstoff der Formel I, der weniger als 20 Gewichtsprozent, vorzugsweise weniger als 10 Gewichtsprozent, insbesondere weniger als 5 Gewichtsprozent des L-Enantiomeren enthält, in Kombination mit üblichen Hilfs- und Trägerstoffen. Die Formulierung für die praktische Anwendung erfolgt nach denselben Methoden und mit den gleichen Zusatzstoffen wie sie für die Racemate bereits bekannt sind. Bevorzugte Formulierungen sind Flüssigformulierungen oder ULV-Formulierungen. Die Formulierungen enthalten die Wirkstoffe vorzugsweise zu 2-95 % je nach Zubereitungsart. Wegen ihrer besseren Wirksamkeit kann mit den erfindungsgemäßen optisch aktiven Wirkstoffen die erforderliche Aufwandmenge pro Flächeneinheit gegenüber den Racematen um bis zu 60 % reduziert werden ; sie liegt im allgemeinen zwischen 0.01-5 kg/ha, vorzugsweise bei 0.05 bis 3 kg/ha.

Herstellungsbeispiele

Beispiel 1

D-(+)-2-[4-(6-Chlor-2-benzthiazolyloxy)-phenoxy]-propionsäure-äthylester

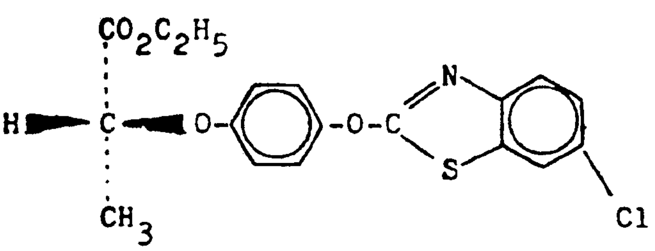

27,8 g (0,1 Mol) 4-(6-Chlor-2-benzthiazolyloxy)-phenol werden mit 16,6 g (0,12 Mol) Kaliumcarbonat in 120 ml Acetonitril 1 1/2 Stunden zum Sieden erhitzt. Anschließend werden 29,9 g (0,11 Mol) L-(Milchsäureäthylester-tosylat, gelöst in 50 ml Acetonitril, innerhalb von 15 Minuten zugetropft. Das Reaktionsgemisch wird nach der Zugabe 5 Stunden am Sieden gehalten, der Salzanteil wird abfiltriert und vom Filtrat Acetonitril Abdestilliert. Der verbleibende Rückstand wird in 200 ml Methylenchlorid aufgenommen, zweimal mit je 100 ml Wasser gewaschen, nach Trocknen über Natriumsulfat wird Methylenchlorid abdestilliert. Der erhaltene Rückstand wird destilliert.

Nach der Destillation erhält man 34,5 g (91.,4 % d.Th.) an überwiegend D-(+) enthaltenden 2-[4-(6-Chlor-2-benzthiazolyloxy)-phenoxy]-propionsäureäthylester mit Fp. 49 °C und $\alpha_D^{20}$ = 9,5° (1m, Chloroform).

Nach Umkristallisieren aus einem Äthanol/Wasser-Gemisch wird das Produkt optisch reiner und hat dann Fp. 51 °C und $\alpha_D^{20}$ = 11° (1 m, Chloroform).

Beispiel 2

D-(+)-Ethyl-2-(4-(6-Chlor-2-benzoxazolyloxy)-phenoxy]-propionat

31,5 g (0,15 Mol) (D+)-Ethyl-2-(4-hydroxyphenoxy)-propionat $[\alpha]_D^{20}$-37,4° (1n $CHCl_3$) werden mit 24,9 g (0,18 Mol) Kaliumcarbonat in 200 ml Acetonitril 1 Stunde zum Sieden erhitzt. Anschließend werden während 45 min 28,2 g (0,15 Mol) 2,6-Dichlorbenzoxazol gelöst in 80 ml Acetonitril zugetropft. Nach 1 Stunde Reaktionszeit werden 0,85 g 2,6-Dichlorbenzoxazol nachgegeben. 1/2 Stunde nach der Zugabe zeigt das Dünnschichtchromatogramm vollständigen Umsatz an. Das Reaktionsgemisch wird abgekühlt und bei 30 °C zur Entfernung des ausgefallenen Salzes abfiltriert, das Filtrat wird eingeengt und bei 80 °C in Vakuum getrocknet. Es hinterbleiben 54,2 g eines ockerfarbenen Feststoffs, der anschließend zweimal unter Zusatz von Tierkohle aus n-Hexan umkristallisiert wird. Man erhält 37,4 g (D+)-Ethyl-2-(4-(6-Chlor-2-benzoxazolyloxy)-phenoxy]-propionat vom Fp. 78,5 °C, der Drehwinkel beträgt $[\alpha]_D^{25}$ : 30° (1n $CHCl_3$).

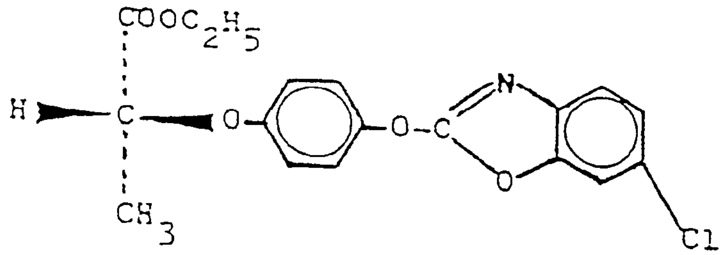

Biologische Beispiele

Die optisch aktiven D-(+)-Verbindungen wurden in Freilandversuchen im Vergleich zu den entsprechenden Racematen im Nachauflaufverfahren gegen verschiedene Schadgräser geprüft. Die Parzellengrößen lagen bei 5 bzw. 10 m²; es wurde mit 3- bis 4-facher Wiederholung gearbeitet. Die Verbindungen wurden als Emulsionskonzentrate formuliert und in verschiedenen Aufwandmengen je ha bei einem Wasseraufwand von 500 l/ha ausgebracht, wobei die Flüssigkeitsmenge, auf 1 ha bezogen, jeweils 500 l betrug.

Die Wirksamkeit der Produkte wurde 30-35 Tage nach der Behandlung mittels « BEA-Schema 1-9 » (Biologische Bundesanstalt, Braunschweig) festgestellt, wobei 1 eine 100%ige Wirkung und 9 keinerlei Wirkung bedeutet. Bei den Kulturpflanzen bedeutet 1 keine schädigung und 9 eine totale schädigung der Pflanzen. Die Kulturpflanzenverträglichkeit wurde außerdem zum ersten Mal bereits eine Woche nach der Behandlung bonitiert.

## Patentansprüche

1. Verbindung der Formel

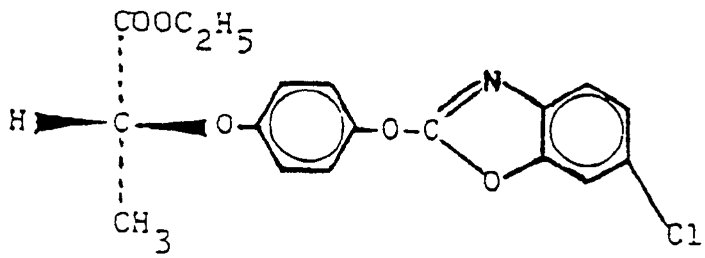

(Ia)

mit einem Gehalt von mindestens 80 % D-Form.

2. Verbindung der Formel

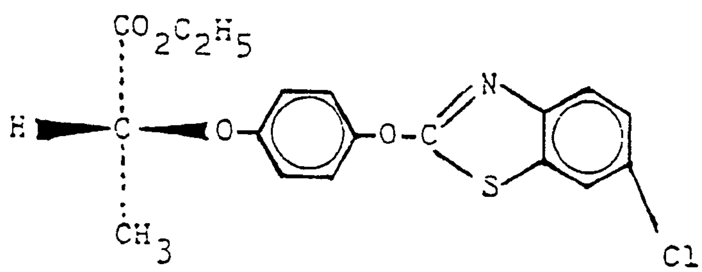

(Ib)

mit einem Gehalt an mindestens 80 % D-Form.

## Claims

1. Compound of the formula

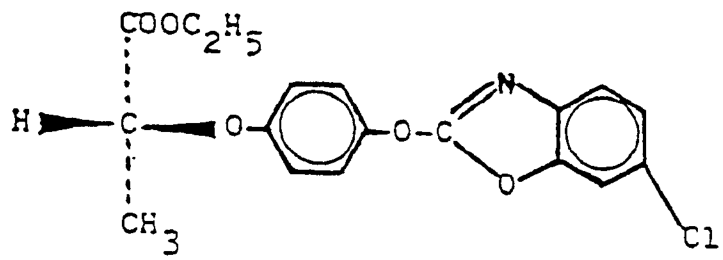

(Ia)

containing at least 80% D-form.

2. Compound of formula

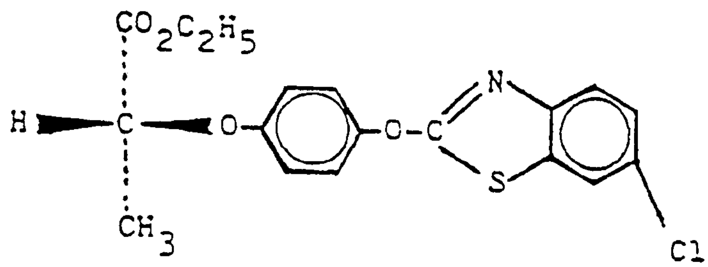

(Ib)

containing at least 80% D-form.

## Revendications

1. Composé de formule

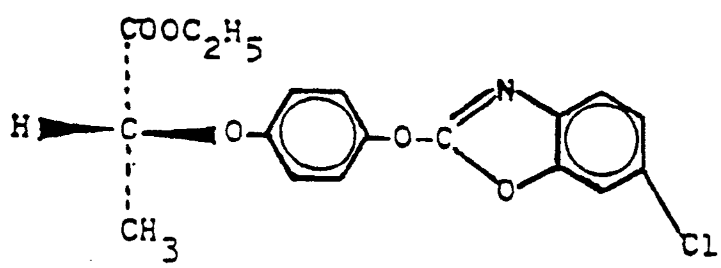

(Ia)

avec une teneur en la forme D d'au moins 80%.

2. Composé de formule

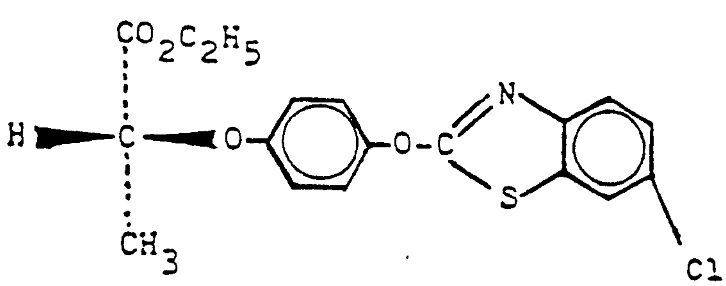

(Ib)

avec une teneur en la forme D d'au moins 80%.